# EUROPEAN PATENT SPECIFICATION

(11) **EP 2 101 769 B1**
(45) Date of publication and mention of the grant of the patent: **24.10.2012**
(21) Application number: 07868760.5
(22) Date of filing: 15.11.2007
(51) Int. Cl.: A61K 31/145, A61K 31/4468, A61K 31/438, A61P 25/32

(54) **METHODS FOR THE TREATMENT OF ALCOHOL ABUSE, ADDICTION AND DEPENDENCY**
VERFAHREN ZUR BEHANDLUNG VON ALKOHOLMISSBRAUCH, -SUCHT UND -ABHÄNGIGKEIT
MÉTHODES DE TRAITEMENT DE L'ABUS D'ALCOOL, DE L'ACCOUTUMANCE ET DE LA DÉPENDANCE

(30) Priority: 28.11.2006 US 861375 P
(43) Date of publication of application: 23.09.2009
(73) Proprietor: Janssen Pharmaceutica, N.V., 2340 Beerse (BE)
(72) Inventor: VAIDYA, Anil, New Jersey 07410 (US)
(74) Representative: Warner, James Alexander
(86) International application number: PCT/US2007/084751
(87) International publication number: WO 2008/067177

(56) References cited:
- WO-A-00/06545
- WO-A-2004/022558

## Description

### FIELD OF THE INVENTION

The present invention is directed to compounds for use in methods for the treatment of alcohol abuse, addiction and / or dependency, alone and in combination with one or more anti-addiction agents.

### BACKGROUND OF THE INVENTION

Alcohol abuse, typically characterized as a maladaptive pattern of alcohol use, leading to clinically significant impairment or distress, is a serious medical and social problem. It has been suggested that agents producing a selective decrease in alcohol drinking in animals, without producing a parallel decrease in water or food intake, are likely to be clinically effective in the treatment of human alcoholism. Daidzin, the active ingredient of the Chinese herb *Radix pureariea (RP),* used as a traditional treatment for "alcohol addiction" in China, fits the profile: it decreases alcohol drinking in the golden hamster, without producing a decrease in water or food intake. In contrast, many drugs, including specific serotonergic agonist (e.g., sertraline) and opiate antagonists (e.g., naloxone and naltrexone), that have been shown to inhibit alcohol consumption in animals have also impaired water or food consumption at the same time. However although atypical antipsychotic have been proposed as possible treatments for substance abuse, there medication may undergo substantial hepatic metabolism in substance abuse patients. The population of patients with hepatic impairment is quite high. Consequently it would be advantageous to treat substance abuse patients with an atypical antipsychotic, which was not significantly metabolized in the liver.

There remains a need to provide an effective treatment for alcohol abuse, addiction and / or dependency.

Battista et al., in U.S. Patent Application 10/656,934 filed September 5, 2003 (published as U.S. Patent Publication 2004/0142,995 on July 22, 2004), which is incorporated by reference herein, disclose hydroxy alkyl substituted 1,3,8-triazaspiro[4.5]decan-4-one derivatives useful in the treatment of disorders and conditions mediated by the ORL-1 G-protein, including anxiety, depression, panic, mania, dementia, bipolar disorder, substance abuse, neuropathic pain, acute pain, chronic pain, migraine, asthma, cough, psychosis, schizophrenia, epilepsy, hypertension, obesity, eating disorders, cravings, diabetes, cardiac arrhythmia, irritable bowel syndrome, Crohn's disease, urinary incontinence, adrenal disorders, attention deficit disorder (ADD), attention deficit hyperactivity disorder (ADHD), Alzheimer's disease, for improved cognition or memory and for mood stabilization.

### SUMMARY OF THE INVENTION

The present invention is directed to a compound of formula (I) or a pharmaceutically acceptable salt thereof for use in a method for the treatment of alcohol abuse, addiction and / or dependency 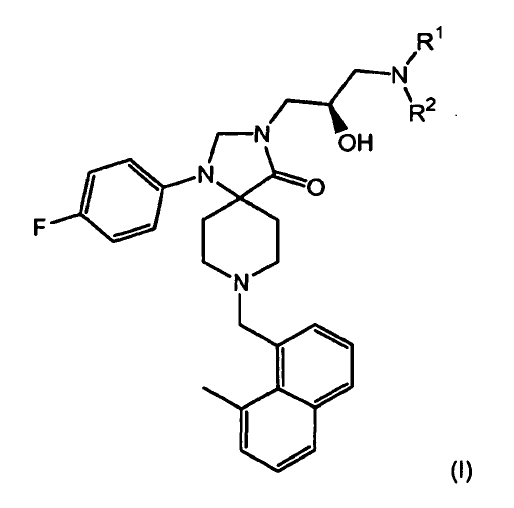 wherein
R¹ and R² are each independently selected from the group consisting of hydrogen and C₁₋₄alkyl.

The present invention is further directed to a compound of formula (I) 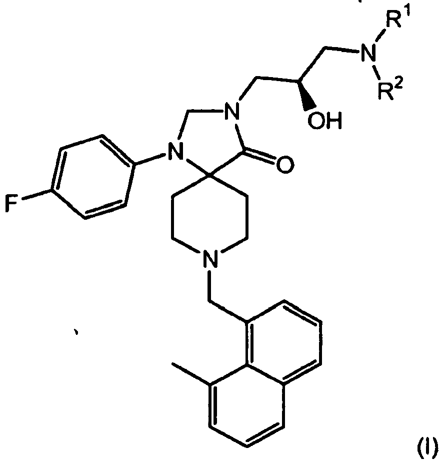 wherein
R¹ and R² are each independently selected from the group consisting of hydrogen and C₁₋₄alkyl;
or a pharmaceutically acceptable salt thereof as a co-therapy with at least one anti-addiction compound , for use in a method of treating alcohol abuse, addiction or dependency.

### DETAILED DESCRIPTION OF THE INVENTION

The present invention is directed to compounds for use in methods for the treatment of alcohol abuse, addiction and / or dependency comprising administering to a subject in need thereof a therapeutically effective amount of a compound of formula (I) 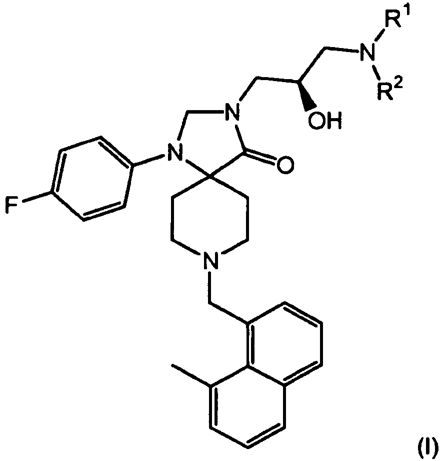 or a pharmaceutically acceptable salt thereof, wherein R¹ and R² are as herein defined, either alone or as co-therapy with one or more anti-addiction agents.

In an embodiment, the present invention is directed to compounds for use in methods for the treatment of alcohol abuse, addiction and / or dependency wherein the compound of formula (I) is the compound of formula (I-A) 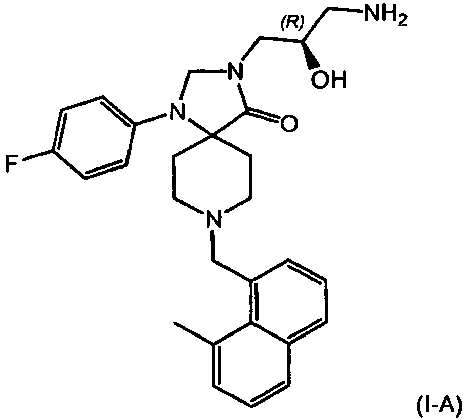 also known as 3-(3-amino-2-(R)-hydroxy-propyl)-1-(4-fluoro-phenyl)-8-(8-methyl-naphthalen-1-ylmethyl)-1,3,8-triazaspiro[4.5]decan-4-one or a pharmaceutically acceptable salt thereof.

In another embodiment, the present invention is directed to compounds for use in methods for the treatment of alcohol abuse, addiction and / or dependency wherein the compound of formula (I) is a crystalline, mono-sulfate salt of the compound of formula (I-A) (preferably a crystalline, anhydrous, non-hygroscopic, mono-sulfate salt of the compound of formula (I-A))

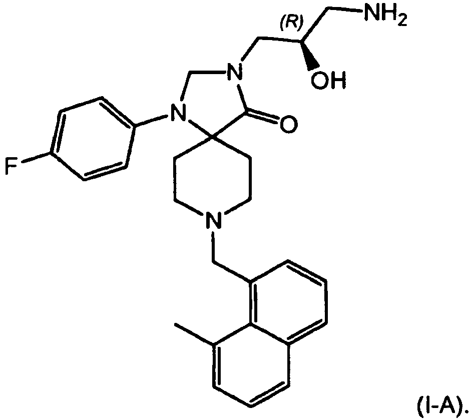

In another embodiment, the present invention is directed to compounds for use in methods for the treatment of alcohol abuse, addiction and / or dependency wherein the compound of formula (I) is a crystalline, mono-sulfate salt of the compound of formula (I-A), as characterized by the position (2θ) and d-spacing of its corresponding XRD peaks.

Representative XRD peaks with a relative intensity greater than or equal to about 10%, measured with a powder X-ray diffractometer, using CuKα radiation, 30mA, 40KV; 1/12° divergence slit, 0.2 receiving slit; scanning from 4 to 30° 2θ at a scan rate of 0.017° 2θ/second and using an aluminum sample holder are as in Table 1 below.

**Table 1**

| **Pos. [°2Th.]** | **d-spacing [Å]** | **Relative Intensity [%]** |
|---|---|---|
| 5.74 | 15.40 | 71.81 |
| 8.12 | 10.89 | 18.52 |
| 9.20 | 9.61 | 45.67 |
| 11.60 | 7.63 | 42.97 |
| 14.74 | 6.01 | 16.18 |
| 14.98 | 5.92 | 16.75 |
| 15.51 | 5.71 | 7.65 |
| 16.30 | 5.44 | 14.14 |
| 16.58 | 5.35 | 9.66 |
| 17.06 | 5.20 | 8.04 |
| 17.28 | 5.13 | 13.65 |
| 18.22 | 4.87 | 100.00 |
| 19.33 | 4.59 | 12.77 |
| 19.60 | 4.53 | 31.86 |
| 20.80 | 4.27 | 30.11 |
| 21.64 | 4.11 | 38.87 |
| 22.04 | 4.03 | 19.09 |

In another embodiment, the present invention is directed to compounds for use in methods for the treatment of alcohol abuse, addiction and / or dependency wherein the compound of formula (I) is a crystalline, mono-sulfate salt of the compound of formula (I-A).

In an embodiment of the present invention, R¹ and R² are each hydrogen. In another embodiment of the present invention, one of R¹ or R² is hydrogen and the other of R¹ or R² is selected from the group consisting of C₁-₄alkyl, preferably a C₁₋₄alkyl selected from the group consisting of methyl, ethyl, isopropyl and t-butyl, more preferably, a C₁₋₄alkyl selected from the group consisting of methyl and ethyl.

As used herein, the term **"C₁₋₄alkyl",** whether used alone or as part of a substituent group, include straight and branched alkyl chain, comprising one to four carbon atoms. For example, alkyl radicals include methyl, ethyl, n-propyl, isopropyl, n-butyl, isobutyl, sec-butyl and t-butyl. Preferably, the C₁₋₄alkyl is selected from the group consisting of methyl, ethyl, isopropyl and t-butyl, more preferably, the C₁₋₄alkyl is selected from the group consisting of methyl and ethyl.

As used herein, unless otherwise noted, the term **"alcohol abuse, addiction and / or dependency"** shall include alcohol related conditions including alcohol abuse, alcohol addiction, alcohol craving (including, but not limited to post-deprivation craving, post-withdrawal craving, relapse craving and binge craving), alcohol dependency, alcohol withdrawal, and related disorders.

As used herein, unless otherwise noted, the term "anti-addiction agent" shall mean any pharmaceutical agent useful for the treatment of alcohol abuse, addiction and / or dependency. More particularly, "anti-addiction agents" include drugs of substitution, drugs of replacement, drugs that block craving, drugs that block or mitigate withdrawal symptoms, drugs which block the pleasurable sensations and rewards of substance abuse.

Suitable examples include naltrexone (also known as REVIA, TREXAN OR VIVTREX), nalmephene, disulfiram (also known as ANTABUSE), acamprosate (also known as CAMPRAL), topiramate (also known as TOPAMAX), risperidone (also known as RISPERDAL), paliperidone, ondansetron (also known as ZORFRAN), selective serotonin reuptake inhibitors (SSRIs such as fluoxetine (also known as PROZAC), sertraline, paroxetine, citalopram, fluvoxamine, and the like), and serorotonin/norepenephrine uptake inhibitors (SNRIs, such as venlafaxine, (also known as EFFEXOR), duloxetine, (also known as CYMBALTA) and the like), and the like. Preferably, the anti-addiction agent is selected from the group consisting of naltrexone, disulfiram and acamprosate.

In an embodiment of the present invention, the anti-addiction agent is selected from the group consisting of naltrexone, nalmephene, disulfiram, acamprosate, topiramate, risperidone, paliperidone, ondansetron, fluoxetine, sertraline, paroxetine, citalopram, fluvoxamine. In another embodiment of the present invention, the anti-addiction agent is naltrexone.

In an embodiment, the present invention is directed to compounds for use in a method for the treatment of alcohol abuse, addiction or dependency, comprising administering to a subject in need thereof a therapeutically effective amount of a compound of formula (I) or pharmaceutically acceptable salt thereof (preferably the compound of formula (I-A) or a pharmaceutically acceptable salt thereof) and one or more anti-addiction agents (preferably one anti-addiction agent.).

In another embodiment of the present invention, the anti-addiction agent is selected from the group consisting of naltrexone, disulfiram, acamprosate, topiramate, risperidone, palieridone and fluoxetine. In another embodiment of the present invention, the anti-addiction agent is an SSRI, preferably an SSRI selected from the group consisting of fluoxetine, sertraline, paroxetine, citalopram and fluvoxamine. In another embodiment of the present invention, the anti-addiction agent is aSNRIs, preferably and SNRI selected from the group consisting of venlafaxine and duloxetine. In another embodiment of the present invention, the anti-addiction agent is selected from the group consisting of naltrexone, disulfiram and acamprosate. In another embodiment of the present invention, the anti-addiction agent is naltrexone.

As used herein, the terms **"co-therapy"** and **"combination therapy"** shall mean treatment of a subject in need thereof by administering one or more compounds of formula (I) in combination with one or more anti-addiction agent(s), wherein the compound(s) of formula (I) and the anti-addiction agent(s) are administered by any suitable means, simultaneously, sequentially, separately or in a single pharmaceutical formulation. Where the compound(s) of formula (I) and the anti-addiction agent(s) are administered in separate dosage forms, the number of dosages administered per day for each compound may be the same or different. The compound(s) of formula (I) and the anti-addiction agent(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) and the anti-addiction agent(s) may be administered according to simultaneous or alternating regimens, at the same or different times during the course of the therapy, concurrently in divided or single forms.

As used herein, unless otherwise noted, the term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to compounds for use as co-therapy or combination therapy, comprising administration of one or more compound(s) of formula (I) and one or more anti-addiction agents, "therapeutically effective amount" shall mean that amount of the combination of agents taken together so that the combined effect elicits the desired biological or medicinal response. For example, the therapeutically effective amount of co-therapy comprising administration of a compound of formula (I) and at least one anti-addiction agent would be the amount of the compound of formula (I) and the amount of the anti-addiction agent that when taken together or sequentially have a combined effect that is therapeutically effective. Further, it will be recognized by one skilled in the art that in the case of co-therapy with a therapeutically effective amount, as in the example above, the amount of the compound of formula (I) and/or the amount of the anti-addiction agent individually may or may not be therapeutically effective.

As used herein, unless otherwise noted, the term "**subject**" as used herein, refers to an animal, preferably a mammal, most preferably a human who has been the object of treatment, observation or experiment. Preferably, the subject has experienced and / or exhibited at least one symptom of the disease or disorder to be treated and / or prevented.

As used herein, the term "composition" is intended to encompass a product comprising the specified ingredients in the specified amounts, as well as any product which results, directly or indirectly, from combinations of the specified ingredients in the specified amounts.

For use in medicine, the salts of the compounds of this invention refer to non-toxic "**pharmaceutically acceptable salts.**" Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesuifonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The present invention further comprises pharmaceutical compositions containing a compound of formula (I) or a compound of formula (I) in combination with one or more anti addiction compounds with a pharmaceutically acceptable carrier for use in a method of treating alcohol abuse, addiction or dependency. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.01-300 mg/kg/day, or any range therein, preferably from about 0.5-10.0 mg/kg/day, more preferably from about 1.0-5.0 mg/kg/day. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation compositions is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The present invention also relates to a pharmaceutical composition comprising any of the compounds as defined herein and a pharmaceutically acceptable carrier, for use in a method of treating alcohol abuse, addiction and / or dependency, The pharmaceutical composition may contain between about 0.1 mg and 1000 mg, or any range therein; preferably about 10 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, com sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

To prepare a pharmaceutical composition of the present invention, a compound of formula (I) as the active ingredient is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Compounds of this invention may be administered in any of the foregoing compositions and according to dosage regimens established in the art whenever treatment of alcohol abuse, addiction and / or dependency is required.

The daily dosage of the products may be varied over a wide range from 0.01 to 5,000 mg per adult human per day, or any range therein. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated. An effective amount of the drug is ordinarily supplied at a dosage level of from about 0.01 mg/kg to about 300 mg/kg of body weight per day, or any range therein. Preferably, the range is from about 0.5 to about 10.0 mg/kg of body weight per day, most preferably, from about 1.0 to about 5.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages. Additionally, one skilled in the art would be able to readily determined recommended dosage levels for known and / or marketed anti-addiction agents by consulting appropriate references such as drug package inserts, FDA guidelines, the Physician's Desk Reference, and the like

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, and the like.

### EXAMPLE 1

### 1-(4-Fluorophenyl)-8-(8-methyl-naphthalen-1-ylmethyl)-1,3,8-triaza-spiro[4.5]-decan-4-one

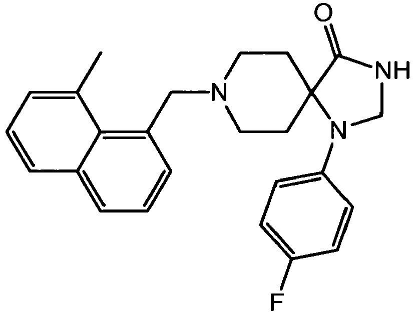

### STEP A: (8-Hydroxymethyl-naphthalen-1-y)-methanol

A 12-L 4-neck flask equipped with a thermocouple, an overhead stirrer, a 2-L addition funnel, and a condenser under N₂ was charged with 1,8-naphthalic anhydride (200 g, 1.0 mol) in toluene (2.5 L) at room temperature. The reaction mixture was agitated while adding DIBAL-H (1.5 M in toluene, 2.664 L, 4 mol) via the addition funnel over 1.5 h. The solution was then heated to 95°C overnight, cooled to 15°C and then slowly diluted with ethyl acetate (2.2 L) and H₂O (2 L) followed by addition of concentrated HCl (320 mL). The resulting suspension was stirred for 30 min at room temperature, filtered, and air dried on the filter for 2 h. The resultant material was in 95% ethanol (1.2 L), stirred at 70°C for 2 h, and filtered to yield a wet solid which was air dried overnight on the filter and then dried at 70°C in a vacuum oven to yield (8-hydroxymethyl-naphthalen-1-yl)-methanol as a solid;
¹H NMR (400 MHz, CD₃OD .δ̇67.85 (2H, dd, *J* = 1.3 and 8.2 Hz), 7.61 (2H, dd, *J* = 1.0 and 7.0 Hz), 7.46-7.42 (2H, m), 5.22 (2H, s), 4.82 (4H, s).

### STEP B: 1H,3H-Benzo[de]isochromene

A 1-L 3-neck flask equipped with an overhead stirrer, a condenser, and a thermocouple was charged with (8-hydroxymethyl-naphthalen-1-yl)-methanol (33.0 g, 0.175 mol), concentrated phosphoric acid (225 mL), and water (5 mL). The reaction mixture was stirred at 140°C for 3 h, cooled to room temperature, diluted with CH₂Cl₂ (800 mL) and transferred to a 2-L separatory funnel. After washing the organic layer with water and saturated NaHCO₃ it was dried over MgSO₄ and evaporated to yield 1*H*,3*H*-Benzo[*de*]isochromene as a solid.
¹H NMR (400 MHz, DMSO-*d₆*): .δ̇ 6.96-6.92 (2H, m), 6.62-6.58 (2H, m), 6.39-6.37 (2H, m), 4.17 (3H, s).

### STEP C: (8-Methyl-naphthalen-1-yl)-methanol (See Tetrahedron, 2000, 56, 8375-8382)

A 3-L 4-neck flask equipped with an overhead stirrer, a thermocouple, a condenser, a nitrogen inlet, and a 1-L addition funnel was charged with potassium (30 g, 0.764 mol) and THF (1 L). The metal suspension was heated to 60°C for 30 min and then stirred to room temperature. To the reaction mixture was then added naphthalene (2 g, 0.015 mol), the suspension was stirred at room temperature for 10 min and then cooled to -20°C to afford a blue suspension. A solution of 1*H*,3*H*-Benzo[de]isochromene (26 g, 0.153 mol) in THF (500 ml) was slowly added via the addition funnel, with addition controlled so that the reaction temperature did not exceed -15°C. After stirring for 5 h at -20°C, the suspension was removed from the cooling bath, warmed with stirring to 0°C, and then allowed to stand without stirring (potassium metal settling). The solution was decanted and the residual potassium was cooled and carefully decomposed with isopropyl alcohol (IPA) under N₂. The decanted solution was carefully treated with water (20 mL) under nitrogen and stirring was continued for 20 min. Additional water and ether were added and the organic layer was separated. The aqueous layer was extracted with CH₂CI₂ and the combined organics were dried over MgS0₄ and condensed *in vacuo* to yield a crude material. The crude material was purified by flash chromatography (7.5/2.5 hexane/EtOAc) to yield 8-methyl-1-napthalenemethanol as a solid.
¹H NMR (300 MHz, DMSO-*d₆*): .δ 7.82-7.80 (1H, m), 7.73-7.69 (1H, m), 7.52-7.50 (1H, m), 7.41-7.32 (3H, m), 5.17 (2H, bs), 3.01 (3H, s).

### STEP D: 8-Methyl-naphthalene-1-carbaldehyde

A 1-L 4-neck equipped with an overhead stirrer, a condenser and a thermocouple was charged with 8-methyl-1-napthalenemethanol (18.5 g, 0.107 mol) in CH₂Cl₂ (500 mL) and stirred at room temperature under N₂. Solid Mn_{(IV})O₂ (61 g, 0.7 mol) was carefully added and the reaction was stirred at room temperature for 3 h, then at 40°C for 6 h and then at room temperature overnight. The reaction mixture was diluted with CH₂Cl₂ (500 mL), filtered and the filtrate was washed with 1N HCl and then dried over MgSO₄. The resulting crude material was purifed using silica gel chromatography (8/2 hexane/ethyl acetate) to yield 8-methyl-naphthalene-1-carbaldehyde as a solid.
¹H NMR (400 MHz, CDCl₃) .δ 10.92 (1H, s), 8.04 (1H, dd, *J* = 1.3 and 8.1 Hz), 7.96 (1H, dd, *J* = 1.4 and 7.1 Hz), 7.82-7.73 (1H, m), 7.55-7.51 (1H, m), 7.49-7.44 (2H, m), 2.82 (3H, s)

### STEP E: 1-(4-Fluoro-phenyl)-8-(8-methyl-naphthalen-1-vymethyl)-1,3,8-triazaspiro[4.5]-decan-4-one.

A 1-L 3-neck flask equipped with an overhead stirrer and a thermocouple was charged with 8-methyl-naphthalene-1-carbaldehyde (13.75 g, 0.08 mol) and 1-(4-fluoro-phenyl)-1,3,8-triaza-spiro[4.5]decan-4-one (21.5 g, 0.085 mol) under N₂ in CH₂Cl₂ (500 mL). After stirring for 20 min, HOAc (1 mL) was added followed by careful addition of solid NaBH(OAc)₃ (33.4 g, 0.157 mol). The mixture was stirred for 16 h at room temperature (suspension becomes a solution). The reaction was then warmed at 50°C for 2 h, cooled down to room temperature and then treated with 0.5 N NaOH (50 mL), stirred for 10 min and then diluted with CH₂Cl₂(100 mL). The organic layer was isolated and dried over MgSO₄. The solvent was evaporated to yield a residue, which was suspended in diethyl ether, stirred for 20 min, filtered and dried in a 60°C vacuum oven to yield 1-(4-fluoro-phenyl)-8-(8-methyl-naphthalen-1-ylmethyl)-1,3,8-triaza-spiro[4.5]-decan-4-one as a white solid.
¹H NMR (400 MHz, CDCl₃) .δ 7.79-7.76 (1H, m), 7.72-7.69 (1H, m), 7.39-7.30 (4H, m), 6.98-6.92 (2H, m), 6.87-6.82 (2H, m), 6.24 (1H, br s), 4.66 (2H, s), 4.01 (2H, s), 3.12 (3H, s), 2.86-2.78 (4H, m), 2.33-2.23 (2H, m), 1.72 (2H, d, *J* = 14.1 Hz);
MS (ES⁺) *m*/*z* 404.2 (M + H)⁺.

### Elemental Analysis:

Calculated: C: 69.26%, H: 7.06%, N: 11.34%, F: 3.91%, H₂O: 1.85%
Measured: C: 68.96%, H: 6.83%, N: 11.38%, F: 4.00%, H₂O: 0.58%

### Example 2

### 1-(4-Fluoro-phenyl)-8-(8-methyl-naphthalen-1-ylmethyl)-3-(S)-oxiranylmethyl-1,3,8-triaza-spiro[4.5]decan-4-one

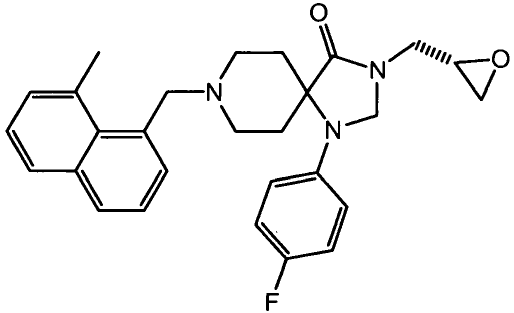

1-(4-Fluoro-phenyl)-8-(8-methyl-naphthalen-1-ylmethyl)-1,3,8-triaza-spiro[4.5]-decan-4-one (2.0 g, 4.95 mmol) was dissolved in N, N-dimethylformamide (25.0 mL). To the reaction mixture was then added at 0°C sodium hydride (60% in mineral oil, 238 mg, 5.94 mmol) under nitrogen atmosphere and the reaction mixture was stirred at 0°C for one hour. To the reaction mixture was then added at 0°C (2R)-(-)-glycidyl-3-nitrobenzenesulfonate (1.54 g, 5.94 mmol). The reaction mixture was stirred at 0°C for one hour, then at room temperature under nitrogen atmosphere for 18 hours and partitioned with water and ethyl acetate. The organic layer was washed with brine, dried with Na₂SO₄, filtered and the solvent evaporated *in vacuo* to yield a crude oil. The crude oil was purified via flash chromatography (2.5% methanol/dichloromethane) to yield the title compound as a foam.
¹H NMR (300 MHz, CDCl₃) δ 7.78-7.76 (1H, m), 7.73-7.69 (1H, m), 7.38-7.31 (4H, m), 6.99-6.91 (2H, m), 6.89-6.84 (2H, m), 4.76 (1H, d, *J* = 4.8 Hz), 4.65 (1H, d, *J* = 4.8 Hz), 4.01 (2H, s), 3.20-3.11 (6H, m), 2.86-2.77 (5H, m), 2.61-2.59 (1H, m), 2.31-2.21 (2H, m), 1.69-1.63 (2H, m)
MS (ES⁺) *m*/*z* 460.2 (M + H)⁺.

### Example 3

### 3-(3-Amino-2-(R)-hydroxy-propyl)-1-(4-fluoro-phenyl)-8-(8-methyl-naphthalen-1-ylmethyl)-1,3,8-triaza-spiro[4.5]decan-4-one

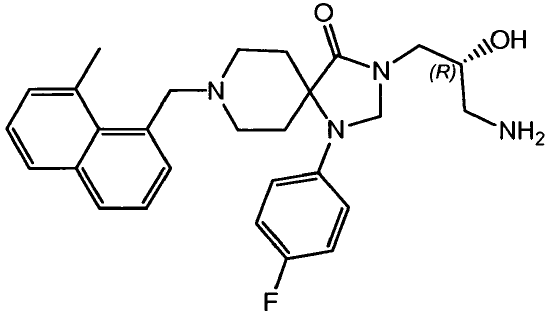

1-(4-Fluoro-phenyl)-8-(8-methyl-naphthalen-1-ylmethyl)-3-(*S*)-oxiranylmethyl-1,3,8-triaza-spiro[4.5]decan-4-one (0.06 g, 0.13 mmol) was dissolved in ethyl alcohol (2 mL) and methyl alcohol (0.4 mL). To the solution was then added concentrated ammonium hydroxide (1 mL) and the reaction mixture was stirred at 40°C for two hours in a pressure flask. The solvent was then evaporated *in vacuo* to yield a crude oil. The crude oil was purified via flash chromatography (5.0% ammonia 2.0 M in methanol /dichloromethane) to yield the title compound as a foam.
¹H NMR (400 MHz, CDCl₃) δ 7.77-7.75 (1H, m), 7.71-7.68 (1H, m), 7.37-7.30 (4H, m), 6.97-6.91 (2H, m), 6.87-6.83 (2H, m), 4.74 (2H, s), 4.0 (2H, s), 3.79-3.74 (1H, m), 3.57-3.52 (1H, m), 3.41-3.36 (1H, m), 3.11 (3H, s), 2.91-2.74 (4H, m), 2.66-2.61 (1H, m), 2.30-2.23 (2H, m), 1.66 (2H, d, *J* = 13.7 Hz)
MS (ES⁺) *m*/*z* 477.1 (M + H)⁺.

### Example 4

### 3-(3-Amino-2-(R)-hydroxy-propyl)-1-(4-fluoro-phenyl)-8-(8-methyl-naphthalen-1-ylmethyl)-1,3,8-triaza-spiro[4.5]decan-4-one, crystalline, anhydrous, mono-sulfate salt

3-(3-Amino-2-(R)-hydroxy-propyl)-1-(4-fluoro-phenyl)-8-(8-methyl-naphthalen-1-ylmethyl)-1,3,8-triaza-spiro[4.5]decan-4-one (50 mg) in ethanol (1 mL) was treated with 1 eq of 18M H₂SO₄ and water (0.2 mL), then heated to dissolve the solids. The resulting solution was then allowed to cool slowly to room temperature overnight. The resulting solid was collected and dried on a filter pad to yield the title compound as a solid.
Initial onset of melt at 196°C, with peaks at 210°C and 224°C

### Example 5

### Preparation of mono-sulfate salt of Compound of Formula (Is) directly from bis-2-keto-L quionic acid salt of Compound of Formula (Is)

3-(3-Amino-2-(R)-hydroxy-propyl)-1-(4-fluoro-phenyl)-8-(8-methyl-naphthalen-1-ylmethyl)-1,3,8-triaza-spiro[4.5]decan-4-one, bis-2-keto-L-gulonic acid salt (10g, 10mmol) and sulfuric acid (1.1 g, 11 mmol) in water (11 mL) were heated to about 75-80°C, and then the resulting solution was treated with ethanol (60g). Upon cooling to 50°C a precipitate was formed. The reaction mixture was cooled to 20-25°C over about 1.5 to 2h and then stirred for about 10-12h. The solids were filtered, washed with ethanol (30g) and dried in a vacuum oven at 60°C to yield the title compound as a solid.

### Example 6

### Recrystallization of mono-sulfate salt of Compound of Formula (Is)

3-(3-Amino-2-(R)-hydroxy-propyl)-1-(4-fluoro-phenyl)-8-(8-methyl-naphthalen-1-ylmethyl)-1,3,8-triaza-spiro[4.5]decan-4-one, bis-2-keto-L-gulonic acid salt (3.3g, 5.74 mmol) in water (95mL) was heated to 100°C. The resulting solution was hot filtered and the filtrate was concentrated under reduced pressure and temperature (50mbar, 60°C) to remove approximately 80g of water. While maintaing the reaction temperature between 60-70°C, ethanol (34mL) was added. After precipitation began the reaction was cooled to 25°C over a period of about 1.5 to 2h and stirring was continued for about 12-14h. The solid was isolated by vacuum filtration and washed with water (2x7mL) and ethanol (9mL). The solids were dried in a vacuum oven at 60°C to yield the title compound as a solid.

### Example 7

### Solid Dosage Forms Comprising Crystalline, Mono-Sulfate Salt of 3-(3-amino-2-(R)-hydroxy-propyl)-1-(4-fluoro-phenyl)-8-(8-methyl-naphthalen-1-ylmethyl)-1,3,8-triaza-spiro[4.5]decan-4-one

Solid, tablet dosage forms comprising the crystalline, mono-sulfate salt of the compound of formula (Is) were prepared with compositions as listed in Table 2. The ingredients were mixed and compressed into tablets, according to known methods. In the table below, the abbreviation BHA stands for butylated hydroxytoluene and the abbreviation BTA stands for butylated hydroxyanisole. PROSOLV HD90® is silicified high density microcrystalline cellulose composed of 98% microcrystalline cellulose and 2% colloidal silicon dioxide. CROSPOVIDONE is a synthetic homopolymer of cross-linked N-vinyl-2-pyrrolidone.

**Table 2: Solid Tablet Dosage Forms**

| | | (mg) | (mg) | (mg) | (mg) | (mg) |
|---|---|---|---|---|---|---|
| Compound (Is) | Active | 1.0 | 5.0 | 25.0 | 100.0 | 250.0 |
| PROSOLV HD90® | Filler | 62.83 | 314.15 | 44.84 | 179.36 | 448.40 |
| CROSPOVIDONE | Disintegrant | 4.0 | 20.0 | 4.0 | 16.0 | 40.0 |
| BHT | Antioxidant | 0.035 | 0.175 | 0.035 | 0.14 | 0.35 |
| BHA | Antioxidant | 0.035 | 0.175 | 0.035 | 0.14 | 0.35 |
| Stearic Acid | Lubricant | 2.1 | 10.5 | 2.29 | 9.16 | 22.9 |

### Example 8

### Alcohol Preferring Rats In Vivo Model

Adult male selectively-bred alcohol preferring rats (N=14) were used. This particular strain of alcohol drinking rat has been characterized and have been widely used to study the effects of different compounds on voluntary alcohol intake (Farren et al., 2000; Rezvani et al, 1990, 1991, 1992a, 1992b, 1999, 2000, 2003; Murphy et al, 1988; Overstreet et al., 2003, 1999; Li and McBride, 1990). Rats were housed individually in approved cages under a constant room temperature of 22 +1°C and 12:12 light-dark cycle (8:00-20:00, dark). Animal were fed Agway Prolab Rat/Mouse/ Hamster 3000 formula and water *ad libitum.*

Alcohol intake was determined using the standard two-bottle choice method utilized in our and other laboratories for many years (Murphy et al. 1988; Rezvani et al. 1990, 1991, 1993, 1995, 1997, Rezvani and Grady, 1994, Rezvani et al., 1993). Animals were first given free access to water in a graduated Richter tube for 2 days. Next, they were given access to only a solution of 10% (v/v) ethanol for 3 consecutive days. During this period animals became accustomed to drinking from Richter tubes and to the taste and pharmacological effects of alcohol. Thereafter, they were given free access to both water and a solution of 10% alcohol for at least 4 consecutive weeks and throughout of the period of study. Rats had free access to food. Water and alcohol intake were recorded at 6 and 24 hour after the treatment, whereas food intake was measured at 24 hour. Animals' body weights were measured the day of the treatment and the day after.

After establishment of stable baseline for alcohol, food, and water intake, rats were administered (gavage) at 9:30 a.m. either with the vehicle or the compound of formula (I) at one of four doses (1,3,10 and 30 mg/kg) using a cross-over design with random assignment. To be able to compare the efficacy of the compound of formula (I) on alcohol intake naltrexone was included as a positive control. The same rats were given an oral dose of 20 mg/kg naltrexone. All animals will receive all treatments in a random order. The interval between treatments was at least 1 week. Alcohol and water intake were recorded 6 and 24 h after the drug administration and food intake was recorded at 24 hr. The volume of the drug administration was 6 ml/kg.

The results at 6 and 24 hours post-dosing are presented in Tables 3 and 4 below, as means ±SEM. Alcohol intake (g/kg) was calculated by multiplying the volume of alcohol consumed in ml by 10% and 0.7893 (ethanol density)/body weight in kg. Alcohol preference, expressed as a percentage, was calculated as follows (volume of alcohol consumed in ml/total fluid intake in ml) x 100 (Rezvani and Grady, 1994; Rezvani et al., 1997). Statistical differences between drug-treated and control groups were determined by using ANOVA with repeated measures. Each treatment was compared with the corresponding vehicle value. The abbreviation NS indicates that the difference in value was not statistically significant.

**Table 3: Effect of Compound (I-A) in Alcohol-Preferring Rats (N=14; 6 hrs post-dosing)**

| **Compound** | **Alcohol Intake (g/kg)** | **Alcohol Preference (%)** | **Total Fluid Intake (mL/kg)** | **Water Intake (mL/kg)** | **Result Type** |
|---|---|---|---|---|---|
| MC (vehicle) | 2.2 ±0.225 | 77.4 ±5.73 | 45.7 ±4..49 | 18.9 ±3.14 | Mean S.E.M. P-value |
| Naltrexone 20 mg/kg p.o. | 1.19 ±0.25 | 46.8 ±7.91 | 39.7 ±6.02 | 12.6 ±2.48 | Mean S.E.M. P-vale |
| | **0.0007** | **0.0003** | NS | **0.0453** | |
| Cmpd (I-A) 1 mg/kg p.o. | 1.85 ±0.234 | 75.1 ±5.60 | 44.7 ±6.60 | 7.36 ±1.67 | Mean S.E.M. P-value |
| | NS | NS | NS | **0.0003** | |
| Cmpd (I-A) 3 mg/kg p.o. | 1.53 ±0.184 | 61.6 ±6.19 | 51.0 ±6.23 | 12.7 ±2.67 | Mean S.E.M. P-value |
| | **0.012** | NS | NS | **0.0477** | |
| Cmpd (I-A) 10 mg/kg p.o. | 1.59 ±0.238 | 62.4 ±6.33 | 46.7 ±4.19 | 9.93 ±2.13 | Mean S.E.M. P-value |
| | **0.034** | NS | NS | **0.0046** | |
| Cmpd (I-A) 30 mg/kg p.o | 0.905 ±0.198 | 46.7 ±8.20 | 38.2 ±6.93 | 11.4 ±2.39 | Mean S.E.M. P-value |
| | **0.0001** | **0.0003** | NS | **0.0174** | |

| | | | | | |
|---|---|---|---|---|---|
| NS = Not Statistically Significant | | | | | |

**Table 4: Effect of Compound (I-A) in Alcohol-Preferring Rats (N=14; 24 hrs post-dosing)**

| **Compound** | **Alcohol Intake (g/kg)** | **Alcohol Preference (%)** | **Total Fluid Intake (mL/kg)** | **Water Intake (mL/kg)** | **Food Intake (g)** | **Result Type** |
|---|---|---|---|---|---|---|
| MC (vehicle) | 5.55 ±0.694 | 81.8 ±5.63 | 105 +12.7 | 37.9 ±5.28 | 21.6 ±1.32 | Mean S.E.M. P-value |
| Naltrexone 20 mg/kg p.o. | 3.64 ±0.545 | 58.8 +6.77 | 118 ±10.8 | 29.9 ±5.21 | 19.1 ±1.34 | Mean S.E.M. |
| | **0.0028** | **0.0008** | NS | NS | NS | P-value |
| Cmpd (I-A) 1 mg/kg p.o. | 4.95 ±0.389 | 80.4 ±4.68 | 104 ±11.1 | 13.1 ±2.26 | 16.3 ±0.773 | Mean S.E.M. P-value |
| | NS | NS | NS | **0.0001** | **0.0012** | |
| Cmpd (I-A) 3 mg/kg p.o. | 4.43 ±0.339 | 68.9 ±4.87 | 120 ±9.23 | 26.2 ±4.56 | 22.4 ±1.07 | Mean S.E.M. P-value |
| | NS | NS | NS | **0.0299** | NS | |
| Cmpd (I-A) 10 mg/kg p.o. | 4.73 ±0.351 | 71.7 ±3.56 | 129 ±9.98 | 24.3 ±3.76 | 22.2 ±1.33 | Mean S.E.M. P-value |
| | NS | NS | **0.040** | **0.0119** | NS | |
| Cmpd (I-A) 30 mg/kg p.o | 3.16 ±0.582 | 50.1 ±7.77 | 123 ±10.3 | 34.0 ±4.73 | 21.5 ±1.26 | Mean S.E.M. P-value |
| | **0.0002** | **0.0001** | NS | NS | NS | |

As shown in Table 1 above, an acute oral administration of 3, 10 and 30 mg/kg of the compound of formula (I-A) significantly reduced alcohol intake at 6 hr when compared with vehicle. As shown in Table 2 above, at 24 hr only the 30 mg/kg dose of the compound of formula (I-A) was statistically significantly (p<0.0002) in reducing alcohol intake. By comparison, naltrexone remained statistically significantly (p<0.0028) in reducing alcohol intake at 24 hr post-dosing. Additionally, at both 6 hours and 24 hours post-dosing, naltrexone and the compound of formula (I-A) at 30 mg/kg reduced alcohol preference. (Other doses of the compound of formula (I-A) did not significantly affect alcohol preference at a statistically significant level).

### Example 9

### Alcohol Preferring Rats - Chronic & Re-administration Studies

Adult male selectively-bred alcohol preferring rats were used in this study. The selectively bred alcohol preferring rats are widely used to study the suppressant effects of compounds on voluntary alcohol intake (Faren et al. 2000; Rezvani et al, 1990, 1991, 1992a, 1992b, 1999, 2000, 2002; 2003; Murphy et al, 1988; Overstreet et al., 1992, 1999; Li and McBride, 1995).

The rats were housed individually under a constant room temperature of 22 ±1°C and 12:12 light-dark cycle (7:00-19:00, dark). Animals were fed Agway Prolab Rat/Mouse/ Hamster 3000 formula and water *ad libitum.*

### Establishment of baseline alcohol intake:

Alcohol intake was determined using a standard two-bottle choice method (Murphy et al. 1988; McBride et al. 1990; Rezvani et al. 1990, 1991, 1993, 1995, 1997, Rezvani et al., 2007a,b; Rezvani and Grady, 1994). Animals were first given free access to water in a graduated Richter tube for 1 day. Next, they were given access to only a solution of 10% (v/v) ethanol for 3 consecutive days. During this period the animals became accustomed to drinking from Richter tubes and to the taste and pharmacological effects of alcohol. Thereafter, the animals were given free access to both water and a solution of 10% alcohol for 4 consecutive weeks and throughout of the study. The animals continued to have free access to food. Water and alcohol intake were recorded at 6 and 24 hour.

### Study A: Chronic Administration:

After establishment of a reliable baseline for alcohol, and water intake, the animals were randomly assigned to a treatment regimen. The animals were administered once a day, via gavage, either vehicle (0.5% methylcellulose) or compound of formula (I-A) (at 30 mg/kg) or naltrexone (at 20 mg/kg) for 14 consecutive days. A group of 6-7 animals was used for each treatment. Alcohol and water intake was recorded at 6 hrs and 24 hrs after administration.

The results are presented as a means +SEM. Alcohol intake (g/kg) was calculated by multiplying the volume of alcohol consumed in ml by 10% and 0.7893 (ethanol density)/body weight in kg. Alcohol preference, expressed as percentage was calculated as (volume of alcohol consumed in ml/total fluid intake in ml) x 100 (Rezvani and Grady, 1994; Rezvani et al., 2007a). Statistical differences between drug-treated and control groups was determined by using ANOVA and Tukey Student's t test for multiple comparison.

The results from Study A provide a measure of whether or not the animals develop tolerance to the suppressant effects of the test compound following a chronic treatment. Results for Study A are as listed in Table 5, 6 and 7, below. In Tables 5-8 below, the abbreviation "(I-A)" stands for the compound of formula (I-A), the abbreviation "Naltx" stands for naltrexone, and the abbreviation "EtOH" stands for ethanol.

**Table 5: Chronic Study Results - Alcohol Intake**

| | **6 Hour Intake Alcohol Intake (G/kg/6 hr)** | | | **24 Hour Intake Alcohol Intake (G/kg/6 hr)** | | |
|---|---|---|---|---|---|---|
| | **Vehicle** | **Naltx (20mg/kg)** | **(I-A) (30mg/kg)** | **Vehicle** | **Naltx (20mg/kg)** | **(I-A) (30mg/kg)** |
| Day 1 | 2.46 | 1.93 | 1.31 | 4.56 | 4.28 | 4.04 |
| Day 2 | 2.96 | 2.08 | 1.54 | 5.19 | 4.75 | 3.71 |
| Day 3 | 3.02 | 2.18 | 1.71 | 5.46 | 5.00 | 3.39 |
| Day 4 | 3.00 | 2.72 | 1.24 | 5.66 | 5.25 | 2.68 |
| Day 5 | 3.38 | 2.85 | 1.54 | 6.45 | 5.30 | 2.97 |
| Day 6 | 2.88 | 2.62 | 1.04 | 5.91 | 5.55 | 2.99 |
| Day 7 | 2.74 | 2.38 | 1.34 | 5.52 | 5.13 | 3.60 |
| Day 8 | 3.18 | 2.56 | 1.63 | 6.12 | 6.07 | 3.97 |
| Day 9 | 3.00 | 2.44 | 1.84 | 6.01 | 6.31 | 3.78 |
| Day 10 | 3.21 | 3.09 | 1.38 | 5.31 | 5.81 | 3.00 |
| Day 11 | 3.18 | 3.10 | 1.17 | 6.29 | 6.13 | 2.86 |
| Day 12 | 2.54 | 2.41 | 1.07 | 5.49 | 5.32 | 3.03 |
| Day 13 | 2.77 | 2.39 | 0.96 | 5.58 | 5.56 | 2.67 |
| Day 14 | 2.92 | 2.65 | 0.55 | 5.86 | 6.01 | 1.91 |

**Table 7: Chronic Study Results - Water Intake**

| | **6 Hour Intake Water Intake (g/kg)** | | | **24 Hour Intake Water Intake (g/kg)** | | |
|---|---|---|---|---|---|---|
| | **Vehicle** | **Naltx (20mg/kg)** | **(I-A) (30mg/kg)** | **Vehicle** | **Naltx (20mg/kg)** | **(I-A) (30mg/kg)** |
| Day 1 | 10 | 12 | 10 | 23 | 28 | 19 |
| Day 2 | 19 | 14 | 19 | 29 | 29 | 32 |
| Day 3 | 11 | 12 | 16 | 26 | 30 | 29 |
| Day 4 | 13 | 9 | 15 | 22 | 21 | 31 |
| Day 5 | 17 | 9 | 28 | 27 | 18 | 37 |
| Day 6 | 10 | 5 | 13 | 26 | 36 | 25 |
| Day 7 | 6 | 5 | 11 | 16 | 14 | 16 |
| Day 8 | 8 | 3 | 12 | 14 | 13 | 23 |
| Day 9 | 8 | 4 | 10 | 17 | 10 | 22 |
| Day 10 | 9 | 4 | 6 | 17 | 13 | 12 |
| Day 11 | 9 | 1 | 9 | 9 | 1 | 9 |
| Day 12 | 7 | 2 | 17 | 7 | 2 | 17 |
| Day 13 | 8 | 4 | 13 | 8 | 4 | 13 |
| Day 14 | 8 | 3 | 16 | 18 | 10 | 28 |

**Table 7: Chronic Study Results - Alcohol Preference**

| | **6 Hour Intake Alcohol Preference (% EtOH)** | | | **24 Hour Intake Alcohol Preference (% EtOH)** | | |
|---|---|---|---|---|---|---|
| | **Vehicle** | **Naltx (20mg/kg)** | **(I-A) (30mg/kg)** | **Vehicle** | **Naltx (20mg/kg)** | **(I-A) (30mg/kg)** |
| Day 1 | 77 | 64 | 62 | 66 | 65 | 72 |
| Day 2 | 70 | 67 | 48 | 70 | 68 | 56 |
| Day 3 | 77 | 70 | 53 | 73 | 68 | 54 |
| Day 4 | 74 | 80 | 52 | 78 | 77 | 50 |
| Day 5 | 71 | 81 | 39 | 75 | 79 | 47 |
| Day 6 | 75 | 86 | 60 | 75 | 72 | 59 |
| Day 7 | 85 | 88 | 65 | 81 | 84 | 76 |
| Day 8 | 84 | 88 | 61 | 84 | 85 | 70 |
| Day 9 | 82 | 89 | 67 | 83 | 89 | 68 |
| Day 10 | 82 | 91 | 72 | 80 | 85 | 73 |
| Day 11 | 82 | 99 | 67 | 90 | 99 | 81 |
| Day 12 | 81 | 95 | 51 | 90 | 97 | 69 |
| Day 13 | 81 | 90 | 45 | 90 | 95 | 67 |
| Day 14 | 82 | 92 | 28 | 82 | 89 | 45 |

As shown in the Tables above, rats treated with Compound of Formula (I-A) showed a decrease in alcohol intake at 6 and 24 hours, over the course of 14 days. The data also indicates that over the 14 days of administration, the animals did not develop tolerance to the alcohol reducing effects of the compound of formula (I-A).

### Study B: Post-deprivation Effect:

Following Study A, the rats were maintained on alcohol for 15 days without drug. On day 16 post-gavage, rats were put on food and water only for 72 hours. On the next day, 45 min before re-exposure to alcohol, the rats were treated with vehicle (0.5% methylcellulose), naltrexone (at 20 mg/kg) or compound of formula (I-A) (at 30-mg/kg). Alcohol intake was then measured at 1, 3, 6 and 24 hr after exposure.

The results are presented as a means. Alcohol intake (g/kg) was calculated by multiplying the volume of alcohol consumed in ml by 10% and 0.7893 (ethanol density)/body weight in kg. Alcohol preference, expressed as percentage was calculated as (volume of alcohol consumed in ml/total fluid intake in ml) x 100 (Rezvani and Grady, 1994; Rezvani et al., 2007a). Statistical differences between drug-treated and control groups was determined by using ANOVA and Tukey Student's t test for multiple comparison.

The results from Study B provide a measure of the effectiveness of the test compound to suppress post-deprivation-induced craving for alcohol. Results for Study B are as listed in Table 8, below.

**Table 8: Study B - Suppression of Post-Deprivation Craving**

| | **1 Hour Post Dosing** | | | |
|---|---|---|---|---|
| **Treatment** | **Alcohol g/kg** | % **EtOH** | **Water g/kg** | **Total fluid** |
| **Vehicle** | 1.21 | 96 | 0.79 | 16.16 |
| **Naltx 20 mg/kg** | 0.48 | 95 | 0.26 | 6.33 |
| **(I-A) 30 mg/kg** | 0.39 | 84 | 0.85 | 5.50 |

| | **3 Hours Post Dosing** | | | |
|---|---|---|---|---|
| **Vehicle** | 1.76 | 95 | 1.13 | 23.45 |
| **Naltx 20 mg/kg** | 1.15 | 94 | 0.88 | 15.46 |
| **(I-A) 30 mg/kg** | 0.72 | 75 | 3.88 | 12.65 |

| | **6 Hours Post Dosing** | | | |
|---|---|---|---|---|
| **Vehicle** | 3.27 | 94 | 3.10 | 44.54 |
| **Naltx 20 mg/kg** | 2.35 | 87 | 4.12 | 33.85 |
| **(I-A) 30 mg/kg** | 1.48 | 72 | 5.78 | 24.68 |

| | **24 Hours Post Dosing** | | | |
|---|---|---|---|---|
| **Vehicle** | 6.63 | 93 | 6.83 | 90.79 |
| **Naltx 20 mg/kg** | 6.47 | 87 | 12.26 | 94.20 |
| **(I-A) 30 mg/kg** | 3.72 | 73 | 14.67 | 62.58 |

As shown in the Table 8 above, rats treated with Compound of Formula (I-A) showed a decrease in alcohol intake and preference at 1, 3, 6 and 24 hours post dosing, suggesting that the compound may be effective at suppressing post-deprivation craving.

## Claims

1. A compound of formula (I) 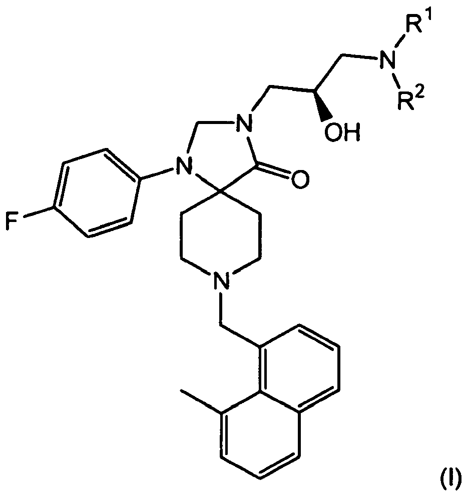 wherein
R¹ and R² are each independently selected from the group consisting of hydrogen and C₁₋₄alkyl;
or a pharmaceutically acceptable salt thereof; alone or as co-therapy with at least one anti-addiction agent, for use in a method of treating alcohol abuse, addiction or dependency,
wherein the co-therapy is one or more compounds of formula (I) in combination with one or more anti-addiction agent(s), wherein the compound(s) of formula (I) and the anti-addiction agent(s) are administered simultaneously, sequentially, separately or in a single pharmaceutical formulation.

2. A compound of formula (I-A) as claimed in Claim 1 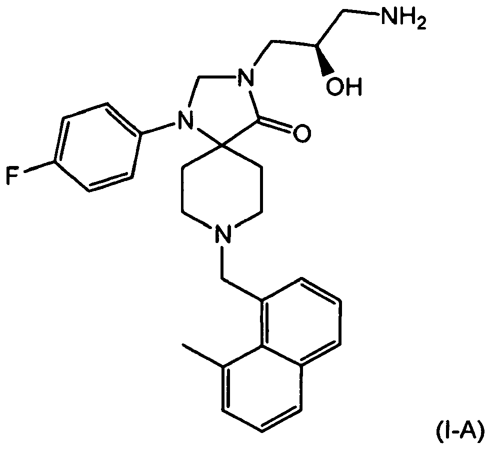 or a pharmaceutically acceptable salt thereof, for use in a method of treating alcohol abuse, addiction or dependency.

3. A compound of formula (I-A) 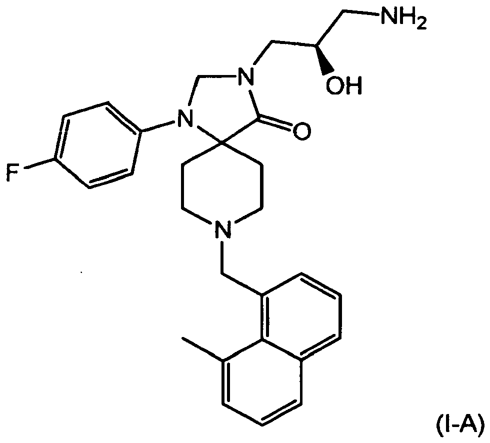 or a pharmaceutically acceptable salt thereof, for use as co-therapy with at least one anti-addiction agent as claimed in Claim 1, for use in a method of treating alcohol abuse, addiction or dependency.

4. A compound for use as claimed in Claim 2 or a compound for use as co-therapy as claimed in Claim 3, wherein the compound of formula (I-A) or pharmaceutically acceptable salt thereof is a crystalline mono-sulfate salt of a compound of formula (I-A).

5. A compound for use as co-therapy as claimed in Claim 3, wherein the anti-addiction agent is selected from the group consisting of a drug of substitution, a drug of replacement, a drug that blocks craving, a drug that blocks or mitigates withdrawal symptoms and a drug which blocks the pleasurable sensations and rewards of substance abuse.

6. A compound for use as co-therapy as claimed in Claim 3, wherein the anti-addiction agent is selected from the group consisting of naltrexone, disulfiram and acamprosate.

7. A compound for use as claimed in Claim 2 or a compound for use as co-therapy as claimed in Claim 3, wherein the alcohol abuse, addiction or dependency is alcohol abuse or alcohol dependency.

8. A compound for use as claimed in Claim 2 or a compound for use as co-therapy as claimed in Claim 3, wherein the alcohol abuse, addiction or dependency is alcohol craving.

9. A compound for use as claimed in Claim 2 or a compound for use as co-therapy as claimed in Claim 3, wherein the alcohol abuse, addiction or dependency is post-withdrawal or relapse craving.

10. A compound for use as claimed in Claim 2 or a compound for use as co-therapy as claimed in Claim 3, wherein the alcohol abuse, addiction or dependency is binge craving.

11. A crystalline, mono-sulfate salt of a compound of formula (I-A) 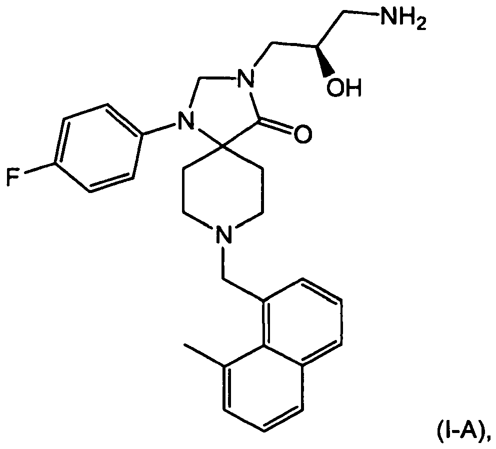 for use as co-therapy with at least one anti-addiction agent as claimed in Claim 1, for use in a method of treating alcohol abuse, addiction or dependency.

12. A compound for use as co-therapy as claimed in Claim 1, Claim 3 or Claim 11, wherein the anti-addiction agent is selected from the group consisting of naltrexone, nalmephene, disulfiram, acamprosate, topiramate, risperidone, paliperidone, ondansetron, fluoxetine, sertraline, paroxetine, citalopram, fluvoxamine, venlafaxine and duloxetine.

13. A compound for use as co-therapy as claimed in Claim 6 or Claim 11, wherein the anti-addiction agent is naltrexone.

14. A compound for use as co-therapy as claimed in Claim 11, wherein the alcohol abuse, addiction or dependency is alcohol abuse, alcohol dependency or alcohol craving.

15. A compound of formula (I-A) 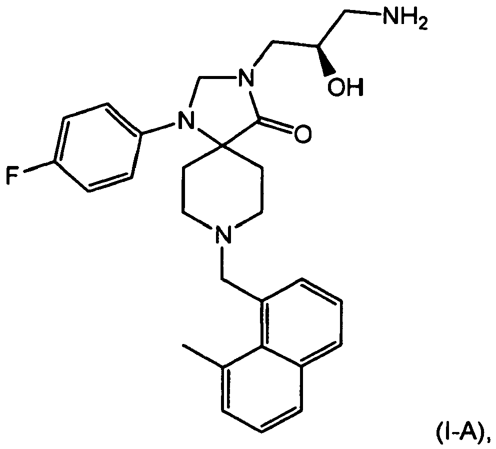 or a pharmaceutically acceptable salt thereof, for use as co-therapy as claimed in Claim 1 with naltrexone.

## Patentansprüche

1. Verbindung der Formel (I) 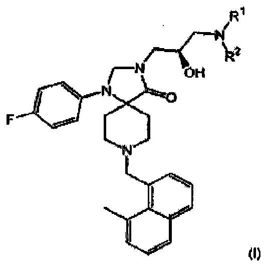 wobei
R¹ und R² jeweils unabhängig voneinander aus der aus Wasserstoff und C₁₋₄-Alkyl bestehenden Gruppe ausgewählt sind,
oder ein pharmazeutisch unbedenkliches Salz davon, alleine oder als Kotherapie mit wenigstens einem anderen Antisuchtmittel, zur Verwendung bei einem Verfahren zur Behandlung von Alkoholmissbrauch, -sucht oder -abhängigkeit,
wobei die Kotherapie aus einer oder mehreren Verbindungen der Formel (I) in Kombination mit einem oder mehreren Antisuchtmitteln besteht, wobei die Verbindung(en) der Formel (I) und das/die Antisuchtmittel gleichzeitig, aufeinanderfolgend, getrennt oder in einer einzelnen pharmazeutischen Formulierung verabreicht werden.

2. Verbindung der Formel (I-A) nach Anspruch 1 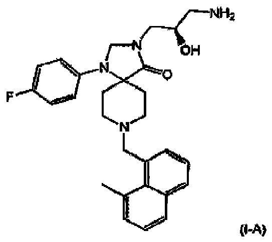 oder ein pharmazeutisch unbedenkliches Salz davon zur Verwendung bei einem Verfahren zur Behandlung von Alkoholmissbrauch, -sucht oder -abhängigkeit.

3. Verbindung der Formel (I-A) 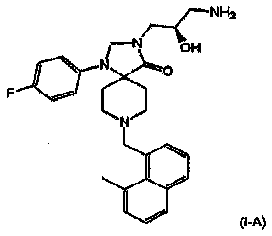 oder ein pharmazeutisch unbedenkliches Salz davon zur Verwendung als Kotherapie mit mindestens einem Antisuchtmittel nach Anspruch 1 zur Verwendung bei einem Verfahren zur Behandlung von Alkoholmissbrauch, -sucht oder -abhängigkeit.

4. Verbindung zur Verwendung nach Anspruch 2 oder Verbindung zur Verwendung als Kotherapie nach Anspruch 3, wobei es sich bei der Verbindung der Formel (I-A) oder dem pharmazeutisch unbedenklichen Salz davon um ein kristallines Monosulfatsalz einer Verbindung der Formel (I-A) handelt.

5. Verbindung zur Verwendung als Kotherapie nach Anspruch 3, wobei das Antisuchtmittel aus der aus einem Substitutionsarzneimittel, einem Ersatzarzneimittel, einem das Verlangen blockierenden Arzneimittel, einem Entzugssymptome blockierenden oder mindernden Arzneimittel und einem die angenehmen Empfindungen und Belohnungen von Substanzmissbrauch blockierenden Arzneimittel bestehenden Gruppe ausgewählt ist.

6. Verbindung zur Verwendung als Kotherapie nach Anspruch 3, wobei das Antisuchtmittel aus der aus Naltrexon, Disulfiram und Acamprosat bestehenden Gruppe ausgewählt ist.

7. Verbindung zur Verwendung nach Anspruch 2 oder Verbindung zur Verwendung als Kotherapie nach Anspruch 3, wobei es sich bei dem Alkoholmissbrauch, der Alkoholsucht bzw. der Alkoholabhängigkeit um Alkoholmissbrauch oder Alkoholabhängigkeit handelt.

8. Verbindung zur Verwendung nach Anspruch 2 oder Verbindung zur Verwendung als Kotherapie nach Anspruch 3, wobei es sich bei dem Alkoholmissbrauch, der Alkoholsucht bzw. der Alkoholabhängigkeit um ein Verlangen nach Alkohol handelt.

9. Verbindung zur Verwendung nach Anspruch 2 oder Verbindung zur Verwendung als Kotherapie nach Anspruch 3, wobei es sich bei dem Alkoholmissbrauch, der Alkoholsucht bzw. der Alkoholabhängigkeit um ein Verlangen nach einem Entzug oder einem Rückfall handelt.

10. Verbindung zur Verwendung nach Anspruch 2 oder Verbindung zur Verwendung als Kotherapie nach Anspruch 3, wobei es sich bei dem Alkoholmissbrauch, der Alkoholsucht bzw. der Alkoholabhängigkeit um ein Verlangen nach einem Alkoholexzess handelt.

11. Kristallines Monosulfatsalz einer Verbindung der Formel (I-A) 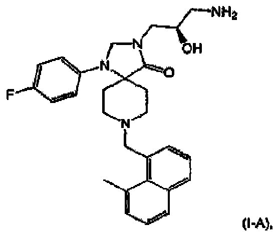 zur Verwendung als Kotherapie mit mindestens einem Antisuchtmittel nach Anspruch 1, zur Verwendung bei einem Verfahren zur Behandlung von Alkoholmissbrauch, -sucht oder -abhängigkelt.

12. Verbindung zur Verwendung als Kotherapie nach Anspruch 1, Anspruch 3 oder Anspruch 11, wobei das Antisuchtmittel aus der aus Naltrexon, Nalmephen, Disulfiram, Acamprosat, Topiramat, Risperidon, Paliperidon, Ondansetron, Fluoxetin, Sertralin, Paroxetin, Citalopram, Fluvoxamin, Venlafaxin und Duloxetin bestehenden Gruppe ausgewählt ist.

13. Verbindung zur Verwendung als Kotherapie nach Anspruch 6 oder Anspruch 11, wobei es sich bei dem Antisuchtmittel um Naltrexon handelt.

14. Verbindung zur Verwendung als Kotherapie nach Anspruch 11, wobei es sich bei dem Alkoholmissbrauch, der Alkoholsucht bzw. der Alkoholabhängigkeit um Alkoholmissbrauch, Alkoholabhängigkeit oder Verlangen nach Alkohol handelt.

15. Verbindung der Formel (I-A) 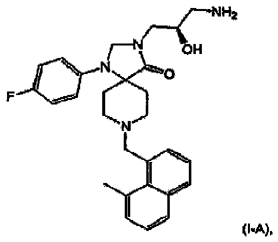 oder ein pharmazeutisch unbedenkliches Salz davon zur Verwendung als Kotherapie nach Anspruch 1 mit Naltrexon.

## Revendications

1. Composé de formule (I) 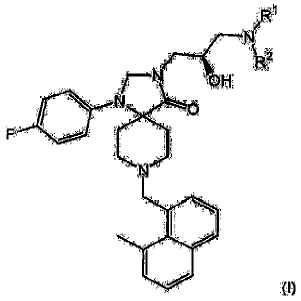 où
chacun des radicaux R¹ et R² est indépendamment choisi dans le groupe constitué par l'atome d'hydrogène et les groupements alkyle en C₁₋₄ ;
ou l'un de ses sels de qualité pharmaceutique ; seul ou en thérapie combinatoire avec au moins un agent anti-addiction, pour emploi dans une méthode de traitement d'une consommation abusive, d'une addiction ou d'une dépendance à l'alcool,
la thérapie combinatoire étant constituée d'un ou de plusieurs composés de formule (I) en combinaison avec un ou plusieurs agents anti-addiction, le ou les composés de formule (I) et le ou les agents anti-addiction étant administrés simultanément, séquentiellement, séparément ou au sein d'une même formule pharmaceutique.

2. Composé de formule (I-A) conforme à la revendication 1 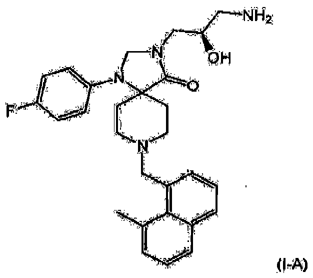 ou l'un de ses sels de qualité pharmaceutique, pour emploi dans une méthode de traitement d'une consommation abusive, d'une addiction ou d'une dépendance à l'alcool.

3. Composé de formule (I-A) 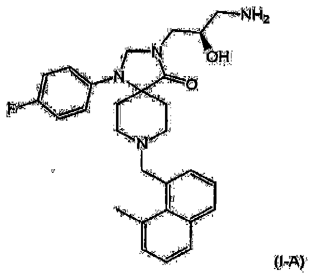 ou l'un de ses sels de qualité pharmaceutique, pour emploi en thérapie combinatoire avec au moins un agent anti-addiction conforme à la revendication 1, pour emploi dans une méthode de traitement d'une consommation abusive, d'une addiction ou d'une dépendance à l'alcool.

4. Composé pour un emploi conforme à la revendication 2 ou composé pour emploi en thérapie combinatoire conforme à la revendication 3, où le composé de formule (I-A) ou son sel de qualité pharmaceutique est un sel de mono-sulfate d'un composé de formule (I-A).

5. Composé pour emploi en thérapie combinatoire conforme à la revendication 3, où l'agent anti-addiction est choisi dans le groupe constitué par un médicament de substitution, un médicament de remplacement, un médicament bloquant l'effet de manque, un médicament bloquant ou réduisant les symptômes de privation et un médicament bloquant les sensations de plaisir et les gratifications de la consommation abusive d'une substance.

6. Composé pour emploi en thérapie combinatoire conforme à la revendication 3, où l'agent anti-addiction est choisi dans le groupe constitué par la naltrexone, le disulfiram et l'acamprosate.

7. Composé pour un emploi conforme à la revendication 2 ou composé pour emploi en thérapie combinatoire conforme à la revendication 3, où la consommation abusive, l'addiction ou la dépendance à l'alcool est une consommation abusive d'alcool ou une dépendance à l'alcool.

8. Composé pour un emploi conforme à la revendication 2 ou composé pour emploi en thérapie combinatoire conforme à la revendication 3, où la consommation abusive, l'addiction ou la dépendance à l'alcool est une sensation de manque d'alcool.

9. Composé conforme à la revendication 2 ou composé pour un emploi conforme à la revendication 3, où la consommation abusive, l'addiction ou la dépendance à l'alcool est une sensation de manque après privation ou après rechute.

10. Composé pour un emploi conforme à la revendication 2 ou composé pour emploi en thérapie combinatoire conforme à la revendication 3, où la consommation abusive, l'addiction ou la dépendance à l'alcool est une sensation de manque d'ébriété excessive.

11. Sel cristallin de mono-sulfate d'un composé de formule (1-A) 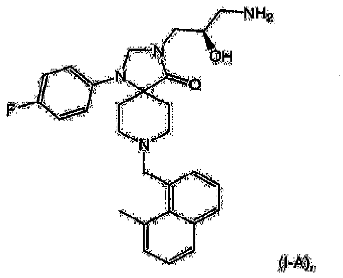 pour emploi en thérapie combinatoire avec au moins un agent anti-addiction conforme à la revendication 1, pour emploi dans une méthode de traitement d'une consommation abusive, d'une addiction ou d'une dépendance à l'alcool.

12. Composé pour emploi en thérapie combinatoire conforme à la revendication 1, à la revendication 3 ou à la revendication 11, où l'agent anti-addiction est choisi dans le groupe constitué par les suivants : naltrexone, nalméphène, disulfiram, acamprosate, topiramate, rispéridone, palipéridone, ondansétron, fluoxétine, sertraline, paroxétine, citalopram, fluvoxamine, venlafaxine et duloxétine.

13. Composé pour emploi en thérapie combinatoire conforme à la revendication 6 ou à la revendication 11, où l'agent anti-addiction est la naltrexone.

14. Composé pour emploi en thérapie combinatoire conforme à la revendication 11, où la consommation abusive, l'addiction ou la dépendance à l'alcool est une consommation abusive d'alcool, une dépendance à l'alcool ou une sensation de manque d'alcool.

15. Composé de formule (I-A) 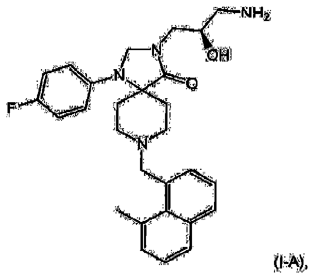 ou l'un de ses sels de qualité pharmaceutique, pour emploi en thérapie combinatoire conforme à la revendication 1 avec la naltrexone.
